# EUROPÄISCHE PATENTSCHRIFT

(11) **EP 0 607 815 B1**
(45) Veröffentlichungstag und Bekanntmachung des Hinweises auf die Patenterteilung: **12.07.2000**
(21) Anmeldenummer: 94100147.1
(22) Anmeldetag: 07.01.1994
(51) Int. Cl.: A61M 39/20

(54) **Zweiteilige Absperrvorrichtung für Schläuche für medizinische Zwecke**
Two part closure for medical tubing
Fermeture en deux parties pour tubes médicaux

(30) Priorität: 19.01.1993 DE 9300616 U
(43) Veröffentlichungstag der Anmeldung: 27.07.1994
(73) Patentinhaber: Büttner, Klaus, D-25336 Klein Nordende (DE)
(72) Erfinder: Büttner, Klaus, D-25336 Klein Nordende (DE)
(74) Vertreter: DIEHL GLAESER HILTL & PARTNER

(56) Entgegenhaltungen:
- DE-C- 3 515 665
- DE-U- 8 906 628
- NL-A- 7 301 986

## Beschreibung

Die Erfindung bezieht sich auf eine zweiteilige Absperrvorrichtung für Schläuche für medizinische Zwecke, aufweisend einen mit dem Schlauchende verbindbaren Stutzen mit Innendurchgang und Außengewinde, einem sog. Luer-Lock negativ, und eine Drehverschlußkappe mit Innengewinde.

In der Medizintechnik werden Leitungen zur Übertragung von Flüssigkeiten benötigt, an die bzw. an deren Endteile besondere Anforderungen gestellt werden, die in der Technik allgemein sonst nicht üblich sind. Es kommt dabei nicht so sehr auf den speziellen Einsatzzweck in der Medizin an, nämlich ob die Leitung bzw. der Schlauch im Zusammenhang mit Dialysegeräten, bei der Bluttransfusion oder für die Medikamentenversorgung eingesetzt wird. Beim Einsatz derartiger extrakorporaler Schläuche oder Leitungen geht es aber immer darum, daß die Leitung im Innenraum und auch an den Endteilen in perfekt sterilisiertem Zustand vorliegt, da nur so eine einwandfreie Verbindung zwischen dem Patienten und dem zugehörigen Gerät geschaffen werden kann. Es ist daher üblich, die Schläuche zusammen mit ihren Verschlußkappen zu sterilisieren und in einer sterilen Verpackung am Einsatzort zur Verfügung zu stellen. Dort kann dann die entsprechende Verbindung hergestellt werden, wobei sich als Vorschrift durchgesetzt hat, daß nur solche Schläuche eingesetzt werden dürfen, bei denen auch die Verschlußkappe nach der Öffnung der Verpackung sich noch an dem vorgesehenen Ort befindet.

In der Praxis kommt es nun häufig vor, daß Schlauchgarnituren deshalb verworfen werden, weil die Drehverschlußkappe nicht mehr auf dem Stutzen des Schlauches aufgeschraubt ist, obgleich alle Teile einwandfrei steril sind. Im Laufe der Sterilisation kann sich nämlich durch die Einwirkung des Sterilisationsmediums die Kappe lösen, wodurch die Sterilisationswirkung selbst nicht beeinträchtigt sondern im Gegenteil vielleicht sogar noch verbessert wird, weil das Sterilisationsmedium mit Sicherheit auch alle Teile im Inneren der Absperrvorrichtung erreichen kann.

In jüngster Zeit setzt sich in zunehmendem Maß die Dampfsterilisation durch, und bei diesem Vorgang wird gerade auf die Drehverschlußkappe einer Absperrvorrichtung derartig eingewirkt, daß diese sich von dem Stutzen lösen will. Die Dampfsterilisation hat jedoch erhebliche Vorteile gegenüber der Gassterilisation, weil bei dieser nie sichergestellt werden kann, daß keine Restgase in der Leitung oder in der Absperrvorrichtung verblieben sind. Im Einsatz können diese Restgase den Patienten erreichen. Bei der Gassterilisation wird vorherrschend Ethylendioxid eingesetzt, ein Gas, das zwar wirksam zum Sterilisieren eingesetzt werden kann, aber äußerst schädlich für den Patienten ist.

Andererseits ist zu bedenken, daß die Drehverschlußkappe vor der Sterilisation nicht stramm auf den Stutzen aufgeschraubt werden kann, weil in diesem Falle jedwede Sterilisationswirkung im Inneren der Absperrvorrichtung so gut wie ausgeschlossen ist. Es muß also zwischen den beiden Teilen, insbesondere zwischen den Konussen aber auch zwischen den Gewindegängen, genügend Freiraum sein, so daß das Sterilisationsmedium vom Innenraum in den Außenraum und umgekehrt gelangen kann, da nur so eine vollständige Sterilisation möglich ist.

Eine der Gattung entsprechende Vorrichtung ist aus der DE-C-35 15 665 bekannt.

Durch die in der DE-C-35 15 665 gezeigten Vorrichtung wird erreicht, dass der Verschlußstopfen nicht ungewollt von dem Anschlußteil gelöst werden kann. Wenn jedoch der Verschlußstopfen gelöst werden soll, um Applikationen vorzunehmen, müssen Axialkräfte angewendet werden, was nicht erwünscht ist.

Der Erfindung befaßt sich mit dem Problem, eine Absperrvorrichtung der eingangs genannten Art so auszugestalten, daß einerseits eine einwandfreie Sterilisation, und zwar vorzugsweise eine Dampfsterilisation, möglich ist, ohne daß andererseits beim Sterilisationsvorgang oder auch nachher die Drehverschlußkappe sich ungewollt vom Stutzen der Absperrvorrichtung lösen kann.

Erreicht wird dies durch die im Kennzeichen des Anspruchs 1 angegebenen Merkmale.

Wesentlich für die Erfindung ist der Zusammenbau von Stutzen und Kappe in der Art, daß einerseits eine geringe Relativbewegung der beiden Teile möglich ist, so daß beispielsweise vom Inneren des Schlauches her der Dampf durch den Stutzen hindurch in das Gewinde und von daher in den Außenraum gelangen kann. Da der Dampf nicht gleichmäßig sondern mehr oder weniger pulsierend eingesetzt wird, kann hierdurch eine einwandfreie Sterilisation aller Innenräume der Absperrvorrichtung sichergestellt werden. Jedoch wird die Relativbewegung der beiden Teile durch Mittel begrenzt, die letzten Endes verhindern, daß die Drehverschlußkappe sich vom Stutzen löst. Zum gewollten Lösen der beiden Teile sind relativ geringe zusätzliche Kräfte erforderlich, die von einer Bedienungsperson ohne weiteres aufgebracht werden, ja sogar nicht einmal gespürt werden. Andererseits ist aber die durch das Sterilisationsmedium ausgeübte Kraft auf die Kappe nicht groß genug, um sie über die Sperre hinweg zu bewegen.

Bei einer besonders bevorzugten Ausführungsform sind die beiden Sperrteile lippenförmig ausgestaltet, wobei die radial nach innen vorstehende Lippe der Drehverschlußkappe radial weiter nach innen hineinragt als die radiale Lippe des Stutzens nach außen hin vorsteht.

Die Erfindung wird nachstehend anhand der Zeichnungen beispielsweise erläutert.
- Fig.1: zeigt eine vergrößerte Querschnittsansicht durch eine Absperrvorrichtung gemäß der Erfindung.
- Figuren 2 bis 4: zeigen weiter vergrößerte Darstellungen des in Fig. 1 innerhalb eines Kreises gezeigten Bereiches.

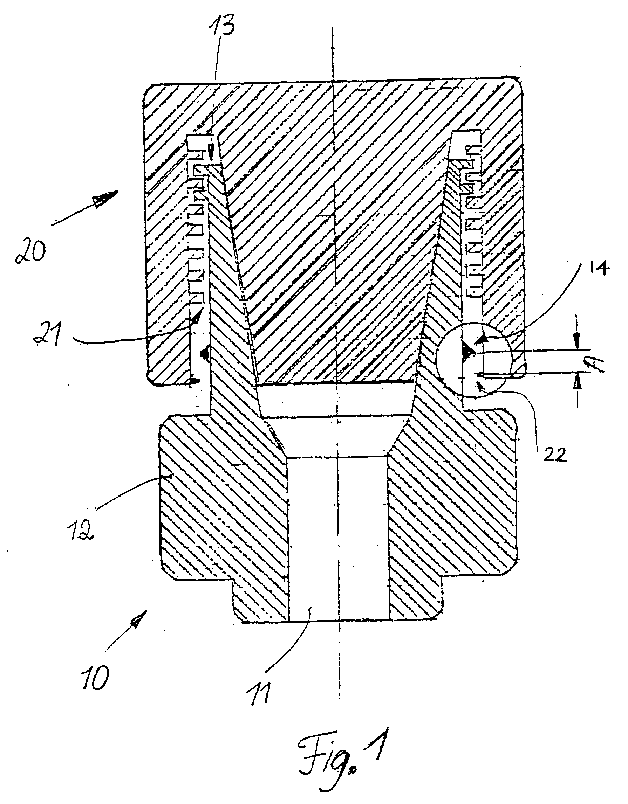

Fig. 1 zeigt einen Stutzen 10 mit einem Innendurchgang 11 und zwei sich diametral gegenüberstehenden Flügeln 12, welche der besseren Handhabung dienen. An seinem äußeren Ende ist der Stutzen 10 mit einem Gewinde 13 und einem Innenkonus ausgebildet, während an dem gegenüberliegenden Ende eine Verbindung zu einem nicht gezeigten Schlauch in bekannter Art und Weise besteht.

Eine Drehverschlußkappe 20 ist mit einem entsprechenden Außenkonus ausgebildet und weist ein Gewinde 21 auf, welches zu dem Gewinde 13 paßt. In der Fig. 1 sind die beiden Teile in Dicht- bzw. Absperrstellung gezeigt. Werden die Teile weiter in die Dichtrichtung zueinander verdreht, so werden, die Konusse eine Absperrung des Innenraums bzw. des Innendurchgangs 11 nach außen hin bewirken. In der Sterilisationsstellung wird die Drehverschlußkappe 20 etwas von dem Stutzen 10 abgedreht, so daß der Innenraum bzw. der Innendurchgang 11 über den Ringspalt zwischen dem Konus und die Zwischenräume in den Gewinden 13 und 21 nach außen hin offen ist.

Bei den in Fig. 1 gezeigten Teilen handelt es sich um Kunststoffteile, beispielsweise aus Polycarbonat, Polypropylen, Polyethylen oder Polyamid. Die räumliche Größe dieser Teile sei dadurch gekennzeichnet, daß für den Durchmesser des Innendurchgangs 11 0,5 bis 2 mm 5 bis 7 mm oder sogar einige cm in Frage kommen, was vom Einsatzzweck abhängt.

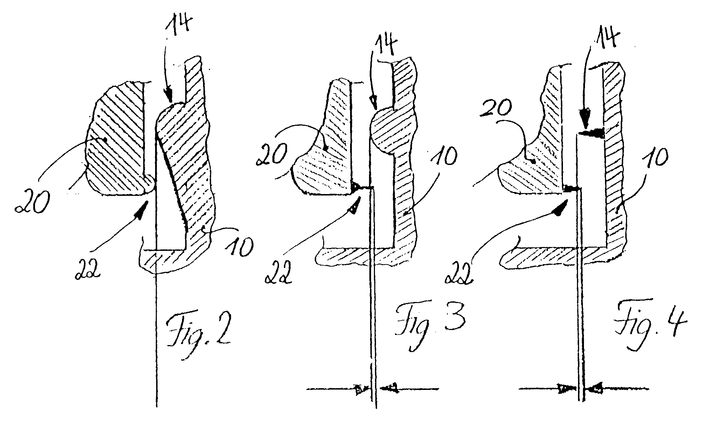

Die Fig. 2 bis 4 zeigen weitere Einzelheiten und lassen wulstförmige Vorsprünge 14 (Fig. 2 und 3) sowie einen lippenförmigen Vorsprung 14 in Fig. 4 erkennen. Diese Lippen bzw. Vorsprünge 14 arbeiten jeweils mit entsprechenden wulstförmigen Vorsprüngen der Drehverschlußkappe 20 zusammen, und zwar mit einem abgerundeten Vorsprung in der Ausgestaltung nach Fig. 2 sowie mit lippenförmigen Vorsprüngen bei den Ausführungsformen nach den Fig. 3 bis 4.

Bei der Ausführungsform nach Fig. 2 fallen die maximalen Radialerstreckungen der Wulste 14 und 22 zusammen, während bei den Ausgestaltungen nach den Fig. 3 und 4 Überlappungen vorliegen, so daß ein gewisser Kraftaufwand erforderlich ist, um die Drehverschlußkappe 20 vom Stutzen 10 abzudrehen. Die hierfür erforderlichen Kräfte liegen jedoch im Bereich von 10 , was ausreichend groß ist, um zu verhindern, daß sich die Drehverschlußkappe 20 zufolge eines Innendruckes durch das Sterilisationsmedium von selbst über diese Sperre hinweg bewegt.

Die Axialerstreckung A, über die sich die beiden Teile 10 und 20 zueinander verdrehen können, ist in Fig. 1 eingezeichnet, und beläuft sich auf etwa A = 1,5 mm.

Die Wulste 14 und 22 erweisen sich auch in anderem Zusammenhang als sehr nützlich, weil nämlich eine zusätzliche Zentrierung der beiden Teile zueinander erfolgt, so daß ein übermäßiges Wackeln der Kappe auf dem Stutzen nicht auftritt.

Bevorzugterweise wird die Wulst an der Kappe in Richtung auf das Gewinde hin versetzt, um das Aufschrauben der Kappe auf den Stutzen zu erleichtern.

Es ist nicht erforderlich, rundumlaufende Wulste einzusetzen, es können Wulste mit Unterbrechungen aber auch einzelne Teilvorsprünge eingesetzt werden.

Als maximale Radialerstreckung der Wulste an der Kappe ist die Höhe der Gewindegänge des Gewindes 21 dem Kappe anzusehen. Eine übermäßige Erstreckung der Wulste würde das Aufschrauben beeinträchtigen oder gar verhindern. In diesem Zusammenhang ist auch das Temperaturspiel der beiden Teile bei der Vulkanisationstemperatur ( ca. 90°C) ebenso wie die thermischen Ausdehnungskoeffizienten der Materialien von Kappe und Stutzen zu berücksichtigen, insbesondere wenn es sich hierbei um Teile aus unterschiedlichen Materialien handelt.

Die Wulste bzw. Lippen können auch als einzelne Hintergreifungsteile ausgestaltet sein.

## Patentansprüche

1. Eine zweiteilige Absperrvorrichtung für Schläuche für medizinische Zwecke, aufweisend einen mit dem Schlauchende verbindbaren Stutzen (10) mit Innendurchgang (11) und Außengewinde (13), einen sog. Luer-Lock negativ, und eine Drehverschlußkappe (20) mit Innengewinde (21), dadurch gekennzeichnet, daß der Stutzen (10) und die Drehverschlußkappe (20) neben der gewindeartigen Verbindung mit einer nur geringe Relativbewegungen zulassenden selbsthemmenden Einrichtung ausgebildet sind, bei der zusammenwirkende Flächen mit erhöhtem Reibungswiderstand in der Form von einzelnen Hintergreifungsteilen oder von wulst- oder lippenförmigen Vorsprüngen (14,22) am Stutzen (10) und an der Drehverschlußkappe (20) vorhanden sind.

2. Vorrichtung nach Anspruch 1, dadurch gekennzeichnet, daß zusammenwirkende Vorsprünge als umlaufende Wülste oder Lippen (14, 22) ausgebildet sind, deren Radialerstreckungen (Fig. 3, Fig. 4) sich gegenseitig überlappen.

3. Vorrichtung nach Anspruch 2, dadurch gekennzeichnet, daß die Wülste oder Lippen (22) der Drehverschlußkappe (20) in der Nähe ihres freien Randes ausgebildet sind.

## Claims

1. A two-part closure arrangement for tubes for medical purposes having a post (10) connectable with the end of the tube, with an interior through passage (11) and external thread (13), a so-called negative Luer lock, and a rotary closure cap (20) with an interior thread (21), characterised in that the post (10) and the rotary closure cap (20) are provided, as well as the thread type connection, with a self-jamming arrangement permitting only small relative movements, in which cooperating surfaces with increased frictional resistance are present on the post (10) and on the rotary closure cap (20) in the form of individual parts engaging behind one another or of bead- or lip-shaped projections (14, 22).

2. Arrangement according to Claim 1, characterised in that cooperating projections are constructed as surrounding beads or lips (14, 22), the radial extents of which mutually overlap (Fig. 3, Fig. 4).

3. Arrangement according to Claim 2, characterised in that the beads or lips (22) of the rotary closure cap (20) are constructed near its free edge.

## Revendications

1. Un dispositif de fermeture en deux parties pour des tubes flexibles à usage médical, présentant un raccord (10) pouvant être relié à l'extrémité du tube flexible, comportant un passage intérieur (11) et un filetage externe (13), ce que l'on désigne par un Luer-Lock négatif, et un chapeau de fermeture tournant (20) avec filetage intérieur (21),
caractérisé en ce que le raccord (10) et le chapeau de fermeture tournant (20), comportent outre la liaison par filetage, un dispositif autobloquant permettant seulement de faibles mouvements relatifs, et qui comprend sur le raccord (10) et sur le chapeau de fermeture tournant (20), des surfaces à action réciproque, comportant une résistance accrue au frottement, sous la forme de pièces indépendantes d'accrochage, ou sous la forme de saillies en forme de bourrelets ou de lèvres (14, 22).

2. Dispositif suivant la revendication 1, caractérisé en ce que des saillies à action réciproque sont réalisées sous la forme de bourrelets ou de lèvres périphériques (14, 22), dont les extensions radiales se recouvrent réciproquement (figure 3, figure 4).

3. Dispositif suivant la revendication 2, caractérisé en ce que les bourrelets ou lèvres (22) du chapeau de fermeture tournant (20) sont réalisés au voisinage de son bord libre.
